Europäisches Patentamt

European Patent Office

Office européen des brevets

(11) Publication number: **0 251 469 B1**

# EUROPEAN PATENT SPECIFICATION

(45) Date of publication of patent specification: **22.06.94** (51) Int. Cl.[5]: **C08F 8/00**, C08C 19/00, C08G 85/00, B01J 31/00

(21) Application number: **87304453.1**

(22) Date of filing: **19.05.87**

(54) **Method for converting substances.**

(30) Priority: **20.05.86 JP 113748/86**

(43) Date of publication of application:
**07.01.88 Bulletin 88/01**

(45) Publication of the grant of the patent:
**22.06.94 Bulletin 94/25**

(84) Designated Contracting States:
**DE ES FR GB**

(56) References cited:
**EP-A- 0 032 610**
**FR-A- 2 132 780**

**CHEMICAL ABSTRACTS, vol. 98, no. 20, 16th May 1983, page 138, abstract no. 163215m, Columbus, Ohio, US; & JP-A-58 00 202 (NIPPON CHEMTECH CONSULTING K.K.) 05-01-1983**

**CHEMICAL ABSTRACTS, vol. 96, no. 6, 8th February 1982, page 146, abstract no. 37643v, Columbus, Ohio, US; & JP-A-81 89 815 (NIPPON CHEMTECH CONSULTING K.K.) 21-07-1981**

(73) Proprietor: **SATAKE TECHNOLOGY RESEARCH AND DEVELOPMENT ASSOCIATES INC.**
**3-3, Koji-machi 5-chome**
**Chiyoda-ku**
**TOKYO(JP)**

Proprietor: **NIPPON PETROCHEMICALS COMPANY, LIMITED**
**3-1, Uchisaiwaicho 1-chome**
**Chiyoda-ku**
**Tokyo(JP)**

(72) Inventor: **Satake, Takeo**
**1-24 Sekido 2-chome**
**Wakayama-shi Wakayama-ken(JP)**
Inventor: **Sato, Norio**
**328-15, Kawato-cho**
**Chiba-shi Chiba-ken(JP)**
Inventor: **Tsuchiya, Shigeharu**
**10-3, Takahama 6-chome**
**Chiba-shi Chiba-ken(JP)**
Inventor: **Hirose, Akira**
**13-4-502, Takasu 1-chome**
**Chiba-shi Chiba-ken(JP)**

**PATENT ABSTRACTS OF JAPAN, vol. 7, no. 14 (C-146)[1159], 20th January 1983; & JP-A-57 171 401 (MARUZEN SEKIYU K.K.) 22-10-1982**

Inventor: **Tsuji, Okitsugu**
**19-8, Wakatake-cho**
**Totsuka-ku**
**Yokohama-shi Kanagawa-ken(JP)**

(74) Representative: **Hardisty, David Robert et al**
**BOULT, WADE & TENNANT**
**27 Furnival Street**
**London EC4A IPO (GB)**

**Description**

BACKGROUND OF THE INVENTION

(1) Field of the Invention

This invention relates to a method for converting substances into other substances. More particularly, the invention relates to the method for converting substances into other substances which comprises the steps of mixing a viscoelastic material having functional groups and being capable of exhibiting spinnability under high shearing stress with a liquid which contains substances to be converted and which have substantially no affinity for said viscoelastic material, and kneading the mixture in a kneader at temperatures in the range of 0 to 100°C under a condition that said mixture exhibits spinnability, thereby converting said substances into other substances by the action of said functional groups.

More specifically, the present invention relates to a method for reacting substances which is characterized in that a viscoelastic material having functional groups that exhibit catalytic activity and having substantially no affinity for a liquid containing reactants is maintained in a condition to exhibit spinnability under high shearing stress, and kneading it while adding thereto a liquid containing reactants, thereby converting the reactants in the liquid into other substances by the acceleration in the reaction of the functional groups.

In another aspect of the invention, it relates to a method for ion-exchange which is characterized in that a viscoelastic material having functional groups that exhibit ion-exchange activity and having substantially no affinity for a liquid containing substances that is subjected to ion-exchange, is maintained in a condition to exhibit spinnability under high shearing stress, and kneading it while adding thereto a liquid containing substances that is subjected to ion-exchange, thereby converting the substances contained in the liquid into other substances.

(2) Description of the Prior Art

Various methods of reaction utilizing the acidic activity or basic activity of certain substances have hitherto been employed widely in the field of chemical industry. To take examples on the use of acidic activity as reaction catalyst, there have been used weak acid catalysts such as carboxylic acids as well as strong acid catalysts of inorganic acids such as hydrochloric acid and sulfuric acid, and organic acid such as sulfonic acid. There have been, however, problems in the corrosion of apparatus and its maintenance because these catalysts having reaction activity are acidic substances.

In place of the acidic substances, acidic ion-exchange resins are also employed as solid catalysts. The acidic ion-exchange resins herein referred to are those generally made by introducing sulfonic groups into styrene-divinylbenzene copolymer or by introducing carboxylic acid groups into the copolymer of styrene, divinylbenzene and acrylic acid or methacrylic acid. As the most of these conventional ion-exchange resins are solid substances, they are made porous by carrying out polymerization methods in order to increase the rate of contact between acidic active groups and substances to be treated, thereby enhancing the reaction efficiency. In this case, however, it cannot be avoided that the contact between reactants and catalytically active radicals becomes difficult because of gradual accumulation of impurities in the fine pore of the resin matrix. It also comes into question in the case of solid resins that the main factor in the reaction rate is the efficiency of solid-liquid contact, therefore, the manner of use of the resin catalyst is limited to some extent.

Furthermore, in order to enhance the release of hydrogen ions from acidic active groups in an ion exchange resin and to improve the efficiency of reaction, small quantity of metal ions such as sodium, potassium, magnesium or calcium ions are added into a reaction system. As the acidic active groups are gradually neutralized, it is necessary to regenerate them.

The high polymers having ion-exchange capacity that are called as ion exchange resins have been used in various industry. They have been hitherto used widely for the purification of water, recovery of metal ions, and in the pharmaceutical industry, for separating, refining and recovering several valuable ingredients. They are also used as solid catalysts for the reactions in which acidic or basic activity is required. Furthermore, the use as carriers for immobilized enzymes in the field of bioreactor is undertaken.

These high polymers are generally solid substances that are composed of the main component of styrene. In order to impart mechanical strength and solvent resistance, a small quantity of divinylbenzene is copolymerized. Ion exchange groups, for example, acidic groups such as sulfonic groups or carboxylic acid groups and basic groups such as amine groups are introduced into the high polymer to give functioned

3

groups having ion exchange capacity. For example, in order to introduce the carboxylic acid groups, a small quantity of acrylic acid or methacrylic acid is copolymerized.

In the case that these solid resins are used for ion exchange, it is important in practical use to maintain the close contact between the ion-exchange groups joined to the surfaces of the solids and substances to be treated contained in a liquid. In view of this fact, the ion exchange resins are commercialized by exercising ingenuity in the copolymerization. In order to attain this purpose, the resin is made porous so as to increase the apparent liquid-holding capacity and the contact area. The strength of the resin is lowered with the rise of porosity, while the contact area and liquid-holding capacity are decreased when the resin is strengthened. Therefore, the improvement in the solid resin naturally has its limit.

In the case of solid resins, the main factor for exchange efficiency, exchange rate and reaction efficiency is dependent on the efficiency of solid-liquid contact, so that the manner of use is restricted. Furthermore, the resins are generally made porous, so that a large number of fine pores exist in resin particles. It has never been avoided that the fine pores are filled up with accumulated impurities which lowers the efficiency of ion exchange.

BRIEF SUMMARY OF THE INVENTION

In view of the foregoing disadvantages in the conventional art, it is the object of the present invention to provide a novel method for converting substances in which the efficiency of substance-exchange is high.

Another object of the present invention is to provide the method for converting substances in which the release of acidic components or basic components does not occur and the corrosion of apparatus is well avoided.

Other objects, features and advantages according to the present invention will become more apparent from the following description.

According to the present invention, the method for converting substances into other substances comprises the steps of mixing a viscoelastic material having functional groups and exhibiting spinnability under high shearing stress with a liquid containing substances to be converted and having substantially no affinity for said viscoelastic material, and kneading the mixture in a kneader at temperatures in the range of 0 to 100°C under a condition that said mixture exhibits spinnability, thereby converting said substances into other substances by the action of said functional groups.

DETAILED DESCRIPTION OF THE INVENTION

The viscoelastic material or a composition of viscoelastic materials (hereinafter simply referred to as "viscoelastic material") having functional groups used in the method of the invention exhibits spinnability when it is kneaded together with a liquid which have substantially no affinity for the viscoelastic material (hereinafter referred to as "non-affinitive liquid") under high shearing stress. The viscoelastic material has a high liquid-holding capacity which means that it can carry or hold therein a large quantity of the non-affinitive liquid. The viscoelastic materials used in the present invention are defined by the following definitions:

The term "spinnability" herein referred to means the property that, when a highly viscous substance which can be deemed like an elastomer in a free condition applied without any force, is kneaded under high shearing stress, it is largely deformed and extended in a form of a thread or threads by its own viscosity. The substance having both elasticity and viscosity and exhibits spinnability when it is kneaded under high shearing stress, is called as "viscoelastic material". Accordingly, the viscoelastic material of the present invention exhibits elasticity in a condition without high shearing stress, while it exhibits both the viscosity and the spinnability under high shearing stress.

In other words, the viscoelastic material or the composition of viscoelastic material in the present invention is a highly viscous fluid at least in a use condition. Accordingly, it can exist in a highly viscous fluid at ordinary temperatures or it may be a solid at ordinary temperatures and it becomes a highly viscous fluid when it is heated to a temperature for operation above its softening point. Furthermore, it may be a highly viscous material which is softened or plasticized by the addition of a softening agent or a plasticizer.

In addition to the fact that the viscoelastic material in the present invention is a highly viscous material under use condition, it exhibits spinnability under high shearing stress. That is, when the viscoelastic material of the present invention is kneaded under high shearing stress, it is largely deformed and extended in a form of threads. The foregoing term "spinnability" herein means the capability of exhibiting such condition.

4

As the viscoelastic material of the present invention is a highly viscous fluid material, it displays the behavior like that of what is called a viscoelastic fluid. The viscoelastic fluid can also exhibit the spinnability (Fundamentals of Fibre Formation, Andrzej Ziabicki, page 13, Wiley-Interscience Publication), however, the viscoelastic material of the present invention exhibits the spinnability when it is subjected to high shearing stress.

The substances which exhibit elasticity without high shearing stress and exhibit viscosity under high shearing stress are exemplified by natural substances such as asphalt, coal tar pitch, rosin, and natural rubber; vinyl resins such as polyvinyl alcohol, partially saponified polyvinyl alcohol, and polyvinyl butyral; synthetic resins produced by addition polymerization such as polybutene, polyisobutylene, butyl rubber, polyisoprene, polybutadiene, styrene-butadiene rubber, polychloroprene rubber, acrylonitrile rubber, atactic polypropylene, polystyrene, polyethylene, polyvinyl chloride, and polyvinyl acetate; and synthetic resins produced by condensation polymerization such as nylon resin and polyester resin. Among these synthetic resin materials, those of low crystallinity or of amorphous nature are preferable. These materials can be used singly or in a mixture or blend of two or more as the viscoelastic material of the present invention. In the case of using a mixture or blend, it is only required that an obtained mixture must be the above specified viscoelastic material that exhibits spinnability under high shearing stress. Specifically preferable viscoelastic materials are polybutene and polymers of isobutylene such as polyisobutylene and their copolymers. The polymers or copolymers of isobutylene are prepared by polymerizing a $C_4$-olefin fraction mainly containing isobutylene in the presence of acid catalyst such as anhydrous aluminum chloride or boron trifluoride.

These materials exhibit elasticity which is regarded apparently as a solid when high shearing stress is not given, however, when they are placed under high shearing stress, they show viscosity and, by kneading them, they are largely deformed with exhibiting spinnability.

The viscoelastic material of the present invention has substantially no affinity for the liquid that is used in the method of the present invention. The wording "has no affinity" herein means the fact that substances are neither compatible nor miscible with each other.

There are two kinds of viscoelastic materials in view of the affinity. One kind is the viscoelastic material which has affinity for non-polar solvents of hydrocarbon oils such as various petroleum fractions and olefin oligomer oil. Among the foregoing substances, the viscoelastic materials of this kind are exemplified by polyolefins such as polybutene and polyisobutylene, and natural rubber.

Contrary to the above, the viscoelastic materials of another kind are those having affinity for polar solvents such as water and alcohols. The viscoelastic materials of this kind are exemplified, among the foregoing ones, by cross-linked polyvinyl alcohol, ethylene-vinyl alcohol copolymer and starch.

In other words, when the viscoelastic material has affinity for non-polar solvents, the non-affinitive liquid used in the method of the present invention is a polar liquid such as water or alcohol. Meanwhile, when the viscoelastic material has affinity for polar solvents, the non-affinitive liquid used in the method of the present invention is a non-polar liquid such as hydrocarbon oil.

The functional groups referred to in the present invention are those capable of converting a substance to be treated that is contained in a non-affinitive liquid, into another substance by means of their activity. The term "convert" used herein includes not only the conversion to convert certain substances themselves or to cause different substances to react producing other substances but also the so-called ion-exchange reaction to convert the ions contained in a non-affinitive liquid into other ions by kneading.

The reaction in this invention is exemplified by the catalytic action that is caused by the conventional acidic or basic ion exchange resins. Furthermore, the reactions in this invention are also exemplified by the catalytic reaction that is caused by the conventional enzymes. For instance, the catalytic reactions caused by acidic functional groups are exemplified by hydrolysis of esters, aldols and acetates; esterification of alcohols with acids; aldol condensation of aldehydes; hydration of olefins such as propylene and butenes into alkanols such as isopropanol and butanol; alkylation of olefins or styrenes to aromatics, that of olefins such as isobutylene with alcohol such as methanol into ethers such as methyl tert-butyl ether and oligomerization. The catalytic reactions caused by basic functional groups are exemplified by the hydrolysis of esters. While, the catalytic reactions caused by enzymatic functional groups are exemplified by the hydrolysis of cane sugar or starch.

Practical examples of the acidic functional groups are carboxylic acid groups, sulfonic acid groups, and phosphoric acid groups. Examples of the basic functional groups are primary to tertiary amino groups and ammonium groups as derivatives of amino groups. These acidic functional groups and basic functional groups may be in the state of free radicals, metal salts, ammonium salts, or other salt forms, or other derivative forms.

5

Furthermore, the functional groups can be those modified ones. For example, the functional group of tertiary amino group is caused to react with a halogenated alkyl or a sulfuric ester by conventional method to obtain a quaternary ammonium salt as a modified functional group. Further, the functional group can be in the form of an immobilized enzyme. For example, by using glutaraldehyde as a cross-linking agent, Schiff base is formed between the amino groups of the viscoelastic material and the amino groups of the protein in several enzymes of hydrolases such as invertase for preparing invert sugar from cane sugar or amylase for hydrolyzing starch, thereby immobilizing the enzymes to the viscoelastic material.

As described above, the viscoelastic materials according to the present invention have functional groups. The functional groups are those joined to a polymer which forms the viscoelastic material as described later. The ratio of the functional groups is at least 0.005 meq per gram and preferably 0.01 meq per gram of viscoelastic material. It is not desirable that the quantity of functional groups is smaller than the above value because the purpose of the present invention cannot be attained.

According to the method of the present invention, the conversion of substances can be carried out by far smaller quantity of the functional groups as compared with the use of conventional ion exchange resin.

The introduction of the functional groups into the viscoelastic materials can be carried out by suitable known methods. In the following, some methods are described with reference to the examples of the introduction of carboxylic acid groups and sulfonic acid groups. For introducing sulfonic acid groups, conventionally known methods for the sulfonation of olefinic hydrocarbons are employed, in which several sulfonic acids such as sulfuric acid, concentrated sulfuric acid, fuming sulfuric acid, and sulfuric acid anhydride; and chlorosulfonic acid are used. The introduction of carboxylic acid groups is carried out by grafting $\alpha,\beta$-unsaturated monocarboxylic acid such as acrylic acid or methacrylic acid; $\alpha,\beta$-unsaturated dicarboxylic acid such as maleic acid, itaconic acid, or citraconic acid, or their anhydrides. When an acid anhydride is grafted, the grafted acid anhydride is decomposed by adding water to obtain dicarboxylic acid. It is possible to introduce functional groups of other kinds by converting these functional groups.

Besides the above methods for introducing functional groups using monomers for grafting, it is also possible to employ the copolymerization using a comonomer such as acrylic acid or vinyl acetate or the polymerization with monomers having functional groups.

The non-affinitive liquids which are used in the present invention by kneading with the viscoelastic materials having functional groups have substantially no affinity for the viscoelastic materials. These non-affinitive liquids besides water are polar organic liquids such as alcohols, ethers and ketones. The alcohols are exemplified by lower alcohols such as methanol, ethanol and isopropanol; and polyhydric alcohols such as ethylene glycol, propylene glycol and glycerol. Preferable ketones are those which have small number of carbon atoms and have water-soluble or substantially hydrophilic property such as acetone and methyl ethyl ketone. Preferable ethers are exemplified by tetrahydrofuran, dioxane and cellosolve. Besides these non-affinitive liquids of oxygen-containing hydrocarbons, nitrogen-containing or sulfur-containing polar solvents such as dimethylformamide, dimethyl sulfoxide and sulfolane which have high solubility or affinity for water, are suitably used. All of these compounds have high polarity and have substantially no solubility or affinity for the viscoelastic materials having functional groups of the present invention.

The above non-affinitive liquids including water are polar liquids. Besides them, the non-affinitive liquids are exemplified by non-polar liquids such as petroleum fractions, olefin oligomer oil, lubricating oil, and motor oil. As far as the polarities are not largely different from each other, a mixture of non-affinitive liquids can also be used.

These non-affinitive liquids can be used by being mixed with the viscoelastic materials of the invention at optional ratio of mixing, as far as the former have substantially no affinity for the latter.

The non-affinitive liquids of the present invention can serve as at least a part of the substances to be converted like the water in the hydrolysis of esters and the hydration of olefins.

It was found out that the viscoelastic material having functional groups and exhibit spinnability when it is kneaded together with the non-affinitive liquid under high shearing stress can contain therein a larger quantity of the non-affinitive liquid as compared with viscoelastic materials having no functional groups.

It is not clear why the viscoelastic material can hold a large quantity of the non-affinitive liquid, however, it is considered that the functional groups of the viscoelastic materials of the present invention are generally polar groups and a large quantity of the non-affinitive liquids are disposed around the polar groups, as a result, much non-affinitive liquids are held uniformly in the viscoelastic materials. When the quantity of a non-affinitive liquid exceeds the liquid holding capacity of a viscoelastic material, separated or free non-affinitive liquid comes out. In other words, when a viscoelastic material and a non-affinitive liquid are kneaded together, the non-affinitive liquid is apparently held within the viscoelastic material. When the non-affinitive liquid is further added to the viscoelastic material more than its holding capacity, free non-affinitive liquid will come out. Accordingly, the kneading of the present invention is done in the coexistence of the viscoelastic material apparently holding the non-affinitive liquid uniformly and excess non-affinitive liquid with exhibiting spinnability. The quantity of a non-affinitive liquid that can be held in a viscoelastic material of the present invention, i.e. the liquid holding capacity varies according to the kind of functional groups, the kind of viscoelastic material having functional groups, and the composition of the viscoelastic material when it is a mixture of viscoelastic materials. However, the quantity of the non-affinitive liquid with regard to the viscoelastic material is generally in the range of 30% by weight to 400% by weight, preferably 40% by weight to 300% by weight and more preferably 40 to 200% by weight.

In order to attain successfully the spinnability and the conversion of substances in the method of the present invention, it is necessary that the non-affinitive liquid is uniformly dispersed and held within the viscoelastic material. In the case that the quantity of non-affinitive liquid is less than 30% by weight, it is not preferable because the concentration of a substance to be contained in the non-affinitive liquid and to be brought into contact with the viscoelastic material becomes low, which results in a low conversion efficiency. On the other hand, when the liquid quantity exceeds 400% by weight, the inversion of phases is caused to occur between the viscoelastic material and the non-affinitive liquid and the viscoelastic material is uniformly dispersed in the non-affinitive liquid, in which the object of the present invention cannot be attained.

In accordance with the invention includes the method for converting substances wherein a viscoelastic material with functional groups having ion exchange capacity is mixed with water containing ions and the mixture is then kneaded in a kneader, thereby replacing ions contained in said water with other ions by the ion exchange of said functional groups.

When the method of the present invention is applied to a conventional field in which the ion exchange resin is used, the contact surfaces are always renewed because the viscoelastic material having functional groups and the non-affinitive liquid are kneaded under the condition of high shearing stress to exhibit spinnability. When the spinnability is exhibited, the viscoelastic materials is deformed in fine threads, therefore, the surface area of the viscoelastic material is made quite large to enhance the apparent active concentration of functional groups. Accordingly, the contact efficiency between the functional groups and the substances to be converted by the functional groups, i.e. the reaction activity or ion exchange activity, can be always maintained very high. In other words, the concentration of a substance to be treated is maintained at a higher level, and therefore, the factor of transfer by diffusion that has been important in the conventional liquid-solid contact system, is out of the question in the present invention. Furthermore, it is to be noted that the non-affinitive liquid held in the viscoelastic material and the excess part of the non-affinitive liquid in a free state are rapidly exchanged to each other by the kneading operation. Therefore, the ion exchange, reaction or other conversion of substances can be attained rapidly. In addition, the efficiency in substance conversion by the functional groups is hardly deteriorated during the kneading.

In order to enhance the liquid holding capacity of the viscoelastic material, it gives sometimes preferable result that viscoelastic materials having different molecular weight distributions are mixed together to obtain a mixture in which the centers of molecular weight distributions are apart from one another. In another measure, the temperature of kneading may be regulated so as to enhance the liquid holding capacity. For example, some amorphous nylon resins show higher liquid holding capacity at higher temperatures while it is reduced at lower temperatures. Furthermore, the liquid holding capacity can be improved by raising the affinity of the functional groups in a viscoelastic material for a non-affinitive liquid, which is carried out by adding and causing to coexist some affinitive organic or inorganic ions such as sodium, potassium, ammonium or carboxylic acid ions.

The carboxylic acids which coexist in the foregoing viscoelastic materials to improve the liquid holding capacity can be polycarboxylic acids such as phthalic acid besides various monocarboxylic acids. The number of carbon atoms in the carboxylic acid can be freely selected. The carboxylic acids used in the present invention may be free carboxylic acids or those which release carboxylic acids by the acidic catalytic activity of the viscoelastic material such as sulfonated isobutylene polymer or copolymer. Exemplified as the compounds which release carboxylic acid are carboxylic acid esters such as monohydric alcohol esters, and dihydric, trihydric and polyhydric alcohol esters such as the esters of ethylene glycol, propylene glycol and glycerol. That is, the carboxylic acid esters may be from the esters of low molecular weight compounds such as methyl acetate to glyceryl esters of higher fatty acids such as animal and vegetable oils. The addition quantities of these carboxylic acids or compounds which release carboxylic acids can be determined in accordance with the viscosities and molecular weights of them.

In the present invention, the quantities of the ions or carboxylic acids added in order to improve the liquid holding capacity, is generally not more than 10% by weight of the viscoelastic material having functional groups.

Furthermore, it is possible to dilute the viscoelastic material by adding liquid materials such as solvents as diluents in order to improve the spinnability during the kneading under high shearing stress. As the diluents, those having high boiling points are preferable so as to avoid the evaporation loss of them during long time kneading operation. More particularly, the diluents are exemplified by kerosene, light oil, turbine oil, and heavy oil which have high boiling points and are miscible with the viscoelastic materials having functional groups of the present invention.

Furthermore, it is possible to mix other viscoelastic materials of the same kind or other high polymers into the viscoelastic material having functional groups as far as the characteristics of the functional groups is not impaired. The other high polymers that can be mixed are not restricted to the viscoelastic materials as specified in the present invention which exhibit spinnability under shear stress. It is only necessary that the mixture obtained exhibits spinnability under high shearing stress as defined in the foregoing paragraph.

The viscoelastic material used in accordance with this invention may be a mixture comprising a polymer having no functional group and a number average molecular weight of 1,000 to 5,000 and a polymer having functional groups and a viscosity average molecular weight of 10,000 to 100,000.

For example, a viscoelastic material having functional groups and a number average molecular weight of 1,000 to 5,000 is mixed with a high polymer (having no functional group) of 10,000 to 1,000,000 in viscosity average molecular weight.

A viscoelastic material, e.g. isobutylene polymer or copolymer, having a number average molecular weight of 1,000 to 5,000 and having functional groups, e.g. sulfonic groups, exhibits a high viscosity. It exhibits spinnability to some extent and it has considerable ion exchange capacity and/or acid-catalytic activity. While, isobutylene polymer or copolymer of 10,000 to 1,000,000 in viscosity average molecular weight is a semi-solid rubber-like substance as it stands, and it has neither ion exchange capacity nor acid-catalytic activity because it has no functional groups. However, when it is mixed with the viscoelastic material having functional groups and a number average molecular weight of 1,000 to 5,000, the mixture shows quite a high conversion efficiency such as ion exchange efficiency or reaction efficiency by the catalytic activity as compared with the case in which the viscoelastic material having functional groups is singly kneaded.

Furthermore, even in the case that viscoelastic materials having functional groups of the same kind, the conversion efficiency can be improved by mixing viscoelastic materials of different molecular weight.

For example, an isobutylene polymer or copolymer having functional groups e.g. sulfonic acid groups and a number average molecular weight of 1,000 to 5,000 and another isobutylene polymer or copolymer having functional groups e.g. sulfonic acid groups and a viscosity average molecular weight of 10,000 to 100,000 are mixed together.

The isobutylene polymer or copolymer having functional groups of sulfonic groups and a number average molecular weight of 1,000 to 5,000 exhibits a high viscosity as it is, and when it is kneaded under high shear stress, it exhibits considerable spinnability and reasonable ion exchange capacity and acid-catalyst activity. Meanwhile, the isobutylene polymer or copolymer having functional groups of sulfonic acid groups and a viscosity average molecular weight of 10,000 to 100,000 is a semi-solid rubber-like substance, and even when it is kneaded under high shear stress, it exhibits spinnability to some extent, however, with the increase in molecular weight, its contact efficiency is not good and the ion exchange capacity and acid-catalyst activity become worse. However, in the case that these are mixed together, particularly higher conversion efficiency such as ion exchange efficiency and reaction efficiency by catalytic activity is exhibited as compared with the case in which a single viscoelastic material is kneaded.

In the method of the present invention, the functional groups are connected to the viscoelastic material and they are not released. Accordingly, not to mention the ion exchange, when it is used, for example, as a catalyst with acidic activity, the operation can be done under almost neutral condition, in which there occurs no fear of the corrosion of apparatus.

The substance to be converted by the method of the present invention is contained in the non-affinitive liquid. This means that the substance to be converted is treated at least in a liquid state and, in fact, it exists as an emulsion, suspension or in a dissolved state.

The substances to be converted are not especially limited as far as they can be converted by the function of the functional groups of the viscoelastic material of the present invention. They are exemplified by esters, acetals and aldols to be hydrolyzed, aldehydes to be subjected to aldol condensation, alcohols and acids such as organic acids to be esterified, olefins to be hydrated, olefins to be alkylated or oligomerized, cane sugar to be inverted, and starch to be hydrolyzed. The amount of these reactants contained in the non-affinitive liquid is not limited as far as it does not impair the foregoing characteristic features of the viscoelastic material of the present invention.

The carboxylic acid esters to be decomposed in the method of the present invention includes monohydric alcohol esters and the esters of dihydric, trihydric or polyhydric alcohols such as ethylene glycol, propylene glycol and glycerol. The carboxylic acids can be polycarboxylic acids such as phthalic acid besides various monocarboxylic acids. The number of carbon atoms in the carboxylic acids can be freely selected. More particularly, they may be from low molecular weight compounds such as methyl acetate to glyceryl esters of higher fatty acids such as natural animal and vegetable oils.

As the ions to be subjected to ion exchange in the ion exchange reaction are exemplified by inorganic ions such as sodium (Na), calcium (Ca) and potassium (K), and organic ions such as amino acids that are subjected to the ion exchange with conventional ion exchange resins.

The kneading according to the present invention may be carried out by cooling, heating or pressurizing in compliance with the purpose of the use. The method of the invention can also be carried out in the presence of an inert gas, if necessary. The suitable temperatures of kneading are in the range of 0 to 100°C. In the case that the method of the invention is carried out at temperatures other than this range, the conversion efficiency is lowered, which is undesirable. With adjusting the temperature within the foregoing range, the spinnability of the viscoelastic materials of the present invention can be varied.

With regard to the operation pressure, it is sufficient that the substance to be treated and the non-affinitive liquid are maintained in liquid state under use conditions. The operation is generally carried out at atmospheric pressure, however, it can be done at an elevated pressure, if desired.

The duration of the operation can be selected within a wide range of time, for example, from 1 minute to 50 hours in accordance with the kind of the conversion method to be carried out.

In order to knead with maintaining the spinnability under high shearing stress, any conventional kneading machines such as a kneader, extruder, and Banbury mixer can be employed in accordance with the purpose of the invention. If necessary, these kneading machines may be equipped with heating or cooling devices. In any event, it is necessary that these kneading machines are those which can exert high shearing stress to the viscoelastic materials in the kneading process and, in fact, it is possible to give necessary high shearing stress by kneading the materials under ordinary conditions with using the above-mentioned kneading machines.

In the following, the present invention will be described in more detail with reference to several examples.

Preparation Example 1

Sulfonation of Polyisobutylene

In 1.5 liter of n-hexane was dissolved 400 g of isobutylene polymer (trademark: Tetrax 3T, made by Nippon Petrochemicals Co., Ltd.; viscosity average molecular weight: 30,000). Maintaining the solution at 50 to 60°C, the polymer was sulfonated by adding 30% fuming sulfuric acid little by little. The quantity of fuming sulfuric acid as $SO_3$ was 5-time moles as much as Tetrax 3T. After that, it was allowed to react for further 2 hours and after the reaction, 1 liter of hexane was again added and the phase of waste sulfuric acid was separated by leaving it to stand still. It was then washed with water until the waste wash water became neutral. The solvent, hexane was then separated by distillation under a reduced pressure to obtain sulfonated isobutylene polymer. The rate of sulfonation was 65% by mole.

Preparation Example 2

Sulfonation of Polybutene

The polymers prepared by polymerizing the $C_4$ fraction in naphtha cracking such as Polybutene HV 300 (trademark, made by Nippon Petrochemicals Co., Ltd.; number average molecular weight: 1,400), Polybutene HV 1900 (trademark, made by the same company; number average molecular weight: 2,700) and Polybutene HV 3000 (trademark, made by the same company; number average molecular weight: 3,700) were sulfonated in the like manner as in Preparation Example 1 to prepare sulfonated Polybutenes. The rates of sulfonation were 72%, 67% and 63% by mole, respectively.

The sulfonated products prepared in the above Preparation Examples 1 and 2 will be hereinafter referred to as "sulfonated Tetrax 3T", "sulfonated Polybutene HV 300", "sulfonated Polybutene HV 1900" and "sulfonated Polybutene HV 3000", respectively.

Preparation Example 3

Sulfonation of Asphalt

In 1.5 liter of cyclohexane was dissolved 400 g of straight asphalt of 60 to 80 in penetration number. Maintaining the solution at 50 to 55°C, the asphalt was sulfonated by adding 30 g of concentrated sulfuric acid little by little. After that, it was allowed to react for further 2 hours and after the reaction, 1 liter of cyclohexane was again added and the phase of waste sulfuric acid was separated by leaving it to stand still. It was then washed with water until the waste wash water became neutral. The solvent, cyclohexane was then separated by distillation under a reduced pressure to obtain sulfonated asphalt. The rate of sulfonation was 3.2% by weight as determined by sulfonic acid content.

Preparation Example 4

Maleic-Modification of Polyisobutylene

To 1 liter autoclave were fed 400 g of isobutylene polymer (trademark: Tetrax 3T, made by Nippon Petrochemicals Co., Ltd.; viscosity average molecular weight: 30,000) and 5 g of maleic anhydride. They were allowed to react for 8 hours at 245°C with stirring. After the reaction, the reaction mixture was cooled and 100 g of water was added thereto and it was maintained at 120 to 130°C so as to convert the acid anhydride into dicarboxylic acid. The obtained mixture was dissolved in 2.5 liter of n-hexane and the phase of water was separated by leaving it to stand still. It was then washed with water until the waste wash water became neutral. The solvent, hexane was then separated by distillation under a reduced pressure to obtain maleic-modified polyisobutylene. The rate of maleic-modification was 72% by mole.

Preparation Example 5

Maleic-Modification of Polybutene

The polymers of isobutylene such as Polybutene HV 300 (trademark, made by Nippon Petrochemicals Co., Ltd.; number average molecular weight: 1,400), Polybutene HV 1900 (trademark, made by the same company; number average molecular weight: 2,700), Polybutene HV 3000 (trademark, made by the same company; number average molecular weight: 3,700) and Tetrax 6T (trademark, made by the same company; viscosity average molecular weight: 60,000) were modified with maleic anhydride in the like manner as in Preparation Example 4 to prepare maleic-modified polyisobutylenes. The rates of maleic-modification were 78%, 75%, 74% and 72% by mole, respectively.

The above maleic-modified products also including that of Preparation Example 4 will be hereinafter referred to as "maleic Tetrax 3T", "maleic Polybutene HV 300", "maleic Polybutene HV 1900", "maleic Polybutene HV 3000" and "maleic Tetrax 6T", respectively.

In the following, the water contents of the viscoelastic materials will be described.

Example 1

To a twin-screw kneader (250 watt, 30-150 rpm) was fed 300 g of maleic Tetrax 3T. With kneading it at a temperature of 40 to 45°C, 6 g of water at a time was added succeedingly and it was observed until free water that was not held in the mixture, was released. The mixture exhibited good spinnability. The water content was determined by the point at which the free water appeared. The water content was 58% by weight relative to maleic Tetrax 3T.

Example 2

In place of 300 g of maleic Tetrax 3T used in Example 1, the following compositions were used.
(A) 150 g of sulfonated Tetrax 3T and 150 g of Tetrax 3T.
(B) 130 g of sulfonated asphalt, 140 g of asphalt and 40 g of kerosene.
(C) 260 g of sulfonated asphalt and 40 g of kerosene.
(D) 150 g of sulfonated asphalt and 150 g of low penetration tar pitch.
(E) 150 g of sulfonated asphalt, 110 g of polyvinyl-butyral and 40 g of light oil.

With regard to the above compositions, water contents were determined in the like manner as in Example 1. All of them exhibited good spinnability and the water contents were 58, 64, 62, 84, and 116%, respectively.

Example 3

A mixture of 200 g of amorphous nylon resin (trademark: T-140, made by Badische Anilin & Soda Fabrik A.G., West Germany) and 30 g of light oil as a softening agent was kneaded at a temperature of 75 to 80°C. By adding water in the like manner as in Example 1, the water content was determined. The water content was 84% by weight and the mixture kneaded together with released water exhibited good spinnability.

Example 4

In place of 300 g of maleic Tetrax 3T used in Example 1, the following compositions were used.
(A) 150 g of maleic Tetrax 3T and 150 g of maleic Polybutene HV 300.
(B) 225 g of maleic Tetrax 3T and 75 g of maleic Polybutene HV 1900.
(C) 90 g of maleic Polybutene HV 300 and 210 g of Tetrax 6T.
(D) 70 g of maleic Polybutene HV 3000, 215 g of Tetrax 6T and 15 g of kerosene as a softening agent.
(E) 90 g of maleic Polybutene HV 300, 162 g of Vistanex MML-80 (trademark, made by Exxon Chem. Corp. and 48 g of kerosene as a softening agent.
(F) 155 g of maleic Tetrax 3T, 75 g of Tetrax 3T, 75 g of Polybutene HV 1900 and 10 g of kerosene as a softening agent.
With regard to the above compositions, water contents were determined in the like manner as in Example 1. All of them exhibited good spinnability and the water contents were 82, 68, 46, 62, 44, and 78%, respectively.

Comparative Example 1

Kneading was carried out in the like manner as in Example 1 by using 300 g of non-modified Tetrax 3T in place of 300 g of the maleic polyisobutylene in Example 1. The mixture exhibited spinnability to some extent, however, the water content was 20% when it was determined in the like manner as in Example 1.
In the following, the ion exchange function of the viscoelastic materials will be described.

Example 5

Ion Exchange Function

To a twin-screw kneader were fed a mixture of 260 g of sulfonated asphalt and 40 g of kerosene and 250 g of water. After kneading them at a temperature of 40 to 45°C, 50 g of saturated saline solution and 110 g of water were added and further kneaded. The concentration of hydrogen ions released into the aqueous phase by ion exchange was determined by acidimetry to examine the ion exchange efficiency. As a result, the ion exchange rates at 2 hours and 4 hours after the addition of the saline solution were 36% and 58%, respectively. Accordingly, it was understood that the viscoelastic material of the present invention has ion exchange capacity.

Example 6

Ion Exchange Function

To a twin-screw kneader were fed a mixture of 150 g of maleic Polybutene HV 3000 and 150 g of maleic Tetrax 3T and 250 g of water and they were kneaded at a temperature of 40 to 45°C, in which the mixture exhibited good spinnability.
After that, 50 g of saturated saline solution and 110 g of water were added to the kneader and further kneaded. The concentration of hydrogen ions released into the aqueous phase by ion exchange was determined by acidimetry to examine the ion exchange efficiency. As a result, the ion exchange rates at 1, 2, 4, and 6 hours after the addition of the saline solution were 18, 34, 53, and 64%, respectively. The ion exchange rates were expressed by the molar percent of the carboxylic acid groups in the polyisobutylene that were neutralized by the sodium ions.

Example 7

Ion Exchange Function

In the like manner as Example 5, ion exchange rates were determined. In this test, 15 g of ethanol was added together with the addition of the saturated saline solution and water. The ion exchange rates at every 1, 2, 4, and 6 hours were 21, 33, 53 and 62%, respectively.

Example 8

Preparation of Viscoelastic Material

Having Ion Exchange Function

A viscoelastic material for ion exchange was prepared by using the composition of sulfonated Tetrax 3T (rate of sulfonation: 50% by mole), non-modified polyisobutylene and kerosene as softening agent.

Table 1

| Composition of Viscoelastic Material | |
|---|---|
| Sulfonated Tetrax 3T | 4.1 kg |
| Polyisobutylene | 4.1 kg |
| Kerosene | 1.24 kg |
| Water | 2.9 kg |

The viscoelastic material of the above composition was put in a kneader of 15 liter kneading capacity and 40 liter volume, and it was kneaded at 40 rpm and 40 to 45°C, in which this viscoelastic material exhibited good spinnability.

Waste Water Treatment by Ion Exchange

Table 2

| Waste Water from Chemical Works | |
|---|---|
| pH | 4.2 |
| C. O. D. | 3140 ppm |
| Suspended Solids | 0 |
| Oil Content | 0 |
| Dry Residue | 10.1% |
| (NaCl used in salting out) | |

The above waste water obtained from a chemical works in Table 2 was diluted and adjusted the salt concentration to about 2100 ppm (as Na) and it was treated by adding it to the foregoing kneaded materials. The results of this treatment are shown in the following Table 3.

Table 3

| Treatment of Waste Water with Ion Exchange Viscoelastic Material | |
| --- | --- |
| Time for Kneading | 1 hour |
| Conc. before Treatment | 2100 ppm |
| Conc. after Treatment | 70 ppm |

As a result of the treatment, it was understood that the ion exchange capacity was 406 mg as Na per kg of sulfonated polyisobutylene.

Comparative Example 2

Kneading was carried out in the like manner as in Example 1 by using singly 300 g of non-modified Tetrax 3T having no functional group. By kneading it with water, good spinnability was exhibited but water content was as low as 24%. After the determination of the water content, a saline solution was added but the pH value of released water was not changed and ion exchange capacity was not exhibited.

Comparative Example 3

Kneading was carried out in the like manner as in Example 1 by using 160 g of non-modified asphalt having no functional group and 40 g of kerosene. By kneading them with water, good spinnability was exhibited but water content was 34%. After the determination of the water content, a saline solution was added but the pH value of released water was not changed and ion exchange capacity was not exhibited.

In the following, the reaction by using the viscoelastic material will be described.

- Hydrolysis -

Example 9

Hydrolysis of Methyl Acetate

To a 1 liter twin-screw kneader were fed 300 g of sulfonated Tetrax 3T and 140 g of water and they were kneaded at a temperature of 40 to 45°C. By this kneading, the mixture exhibited relatively good spinnability.

Then, 15 g of methyl acetate and 160 g of water were added to the kneader and further kneaded. The decrease of methyl acetate in the water phase and the increase of methanol produced by hydrolysis were determined so as to examine the rate of hydrolysis. As a result, the rates of hydrolysis at 2, 4, 8 and 12 hours after the addition of the methyl acetate were 6, 12, 17 and 18% by mole, respectively.

Example 10

Hydrolysis of Methyl Acetate

In place of 300 g of the sulfonated Tetrax 3T in Example 9, a mixture of 150 g of sulfonated Tetrax 3T and 150 g of sulfonated Polybutene HV 3000 was used to hydrolyze methyl acetate in the like manner as Example 9. When the mixture and water were mixed together, good spinnability was exhibited like Example 9. The rates of hydrolysis at 2, 4, 8 and 12 hours after the start of reaction were 30, 50, 70 and 78% by mole, respectively.

Example 11

Hydrolysis of Methyl Acetate

In place of 300 g of the sulfonated Tetrax 3T in Example 9, a mixture of 225 g of sulfonated Tetrax 3T and 75 g of sulfonated Polybutene HV 1900 in Experiment A and a mixture of 210 g of Tetrax 6T, 210 g of Tetrax 6T and 90 g of sulfonated Polybutene HV 300 in Experiment B were used to hydrolyze methyl acetate in the like manner as Example 9. The rates of hydrolysis at 2, 4, 8 and 12 hours after the start of reaction are shown in the following Table 4.

Table 4

| Time (hrs) | 2 | 4 | 8 | 12 |
|---|---|---|---|---|
| Experiment A | 46 | 73 | 87 | 92 |
| Experiment B | 43 | 62 | 80 | 86 |

Example 12

Hydrolysis of Methyl Acetate

In place of 300 g of the sulfonated Tetrax 3T in Example 9, a mixture of 215 g of sulfonated Tetrax 6T and 70 g of sulfonated Polybutene HV 3000 and 15 g of kerosene as a softening agent were used to hydrolyze methyl acetate in the like manner as Example 9. When the mixture and water were mixed together, good spinnability was exhibited like Example 9. The rates of hydrolysis at 2, 4, 8 and 12 hours after the start of reaction were 41, 65, 79 and 83% by mole, respectively.

Example 13

Hydrolysis of Methyl Stearate

To a 1 liter twin-screw kneader were fed a mixture of 150 g of sulfonated Tetrax 3T and 150 g of sulfonated Polybutene HV 3000 and 150 g of water and they were kneaded at a temperature of 40 to 45°C. By this kneading, the mixture exhibited good spinnability.

Then, 15 g of methyl stearate and 200 g of water were added to the kneader and further kneaded. The increase of methanol in the water phase produced by the hydrolysis was determined so as to examine the rate of hydrolysis. As a result, the rates of hydrolysis at 2, 4, 8 and 12 hours after the addition of the methyl stearate were 3, 11, 40 and 53% by mole, respectively.

Example 14

Hydrolysis of Methyl Laurate

Methyl laurate was hydrolyzed in the like manner as Example 13 using the same in place of the methyl stearate. As a result, the rates of hydrolysis at 2, 4, 8 and 12 hours after the addition of the methyl laurate were 8, 18, 35 and 48% by mole, respectively.

Example 15

Hydrolysis of Dimethyl Phthalate

Dimethyl phthalate was hydrolyzed in the like manner as Example 13 using the same in place of the methyl stearate. As a result, the rates of hydrolysis at 2, 4, 8 and 12 hours after the addition of the dimethyl phthalate were 22, 41, 61 and 70% by mole, respectively.

Example 16

Hydrolysis of Rice Bran Oil

To a 1 liter twin-screw kneader were fed a mixture of 150 g of sulfonated Tetrax 3T and 150 g of sulfonated Polybutene HV 3000 and 150 g of water and they were kneaded at a temperature of 40 to 45°C. By this kneading, the mixture exhibited good spinnability.

Then, 15 g of rice bran oil and 200 g of water were added to the kneader and further kneaded. The increase of glycerol in the water phase produced by the hydrolysis was determined so as to examine the rate of hydrolysis. As a result, the rates of hydrolysis at 2, 4, 8 and 12 hours after the addition of the rice bran oil were 3, 7, 22 and 32% by mole, respectively.

Example 17

Hydrolysis of Methyl Acetate

To a 1 liter twin-screw kneader were fed 270 g of sulfonated asphalt, 30 g of kerosene and 140 g of water and they were kneaded at a temperature of 40 to 45°C. By this kneading, the mixture exhibited good spinnability.

Then, 15 g of methyl acetate and 160 g of water were added to the kneader and further kneaded. The decrease of the methyl acetate and the increase of methanol in the water phase produced by the hydrolysis were determined so as to examine the rate of hydrolysis. As a result, the rates of hydrolysis at 2, 4, 8 and 12 hours after the addition of the methyl acetate were 11, 26, 32 and 34% by mole, respectively.

Example 18

Hydrolysis of Methyl Acetate

In place of the sulfonated asphalt and kerosene used in Example 17, 300 g of sulfonated Tetrax 3T was used to hydrolyze methyl acetate. When water was mixed together, good spinnability was exhibited like Example 17. The rates of hydrolysis at 2, 4, 8 and 12 hours after the start of reaction were 6, 12 17 and 18% by mole, respectively.

Example 19

Hydrolysis of Methyl Stearate

To a 1 liter twin-screw kneader were fed 130 g of sulfonated asphalt, 140 g of asphalt, 30 g of kerosene and 150 g of water and they were kneaded at a temperature of 45 to 50°C. By this kneading, the mixture exhibited good spinnability.

Then, 15 g of methyl stearate and 200 g of water were added to the kneader and further kneaded. The increase of methanol in the water phase produced by the hydrolysis was determined so as to examine the rate of hydrolysis. As a result, the rates of hydrolysis at 2, 4, 8 and 12 hours after the addition of the methyl stearate were 4, 13, 37 and 49% by mole, respectively.

Example 20

Hydrolysis of Dimethyl Phthalate

In place of the methyl stearate, dimethyl phthalate was used in the like manner as Example 19 to hydrolyze the dimethyl phthalate. As a result, the rate of hydrolysis at 8 hours after the addition of the dimethyl phthalate was 55% by mole.

Example 21

Hydrolysis of Methyl Acetate

In place of the sulfonated asphalt and kerosene used in Example 19, the hydrolysis of methyl acetate was carried out in the like manner as in Example 19 using the following mixtures:

(A) A mixture of 150 g of sulfonated asphalt and 150 g of low penetration tar pitch.
(B) A mixture of 150 g of sulfonated asphalt, 110 g of polyvinylbutyral and 40 g of light oil.
The rates of hydrolysis at 8 hours after the start of hydrolysis were 28 and 22% by mole, respectively.

Example 22

Test for Inversion of Cane Sugar

A viscoelastic material having the composition shown in Table 1 of Example 8 was fed into a kneader of 15 liter in kneading capacity and 40 liter volume and it was kneaded at 40 rpm and 40 to 45°C, in which this viscoelastic material exhibited good spinnability.

A cane sugar solution of 60° Brix was added to the viscoelastic material and they were kneaded to examine the inversion of the cane sugar. The results are shown in Table 5.

Table 5

| Inversion of Cane Sugar | | |
|---|---|---|
| Duration of Reaction | pH | Rate of Inversion |
| 0.5 hours | 3.46 | 0.7% |
| 1.5 hours | 2.78 | 2.7% |
| 2.5 hours | 2.65 | 6.2% |
| 3.5 hours | 2.62 | 12.9% |
| 4.5 hours | 2.65 | 16.0% |
| 5.5 hours | 2.62 | 17.9% |

- Esterification -

Example 23

Reaction for Esterification

To a twin-screw kneader were fed 300 g of sulfonated Tetrax 3T and 140 g of water and they were kneaded at 45°C. Meanwhile, 13 g of methanol and 12 g of acetic acid were dissolved in 100 g of water and the solution was added to the kneader. Four hours after the addition, the methyl acetate contained in the released aqueous solution was determined to obtain a value of 37% by mole in the rate of esterification.

- Aldol Reaction -

Example 24

Aldol Reaction of Acetaldehyde

In the like manner as in Example 23, water and sulfonated Tetrax 3T were kneaded and 140 g of 10 wt% aqueous solution of acetaldehyde was added to the mixture and they were kneaded. Four hours after the addition, the aldol contained in the released aqueous solution was determined to obtain a value of 34% by mole in reaction rate.

- Effect of Mixing Viscoelastic Materials -

The effect of mixing viscoelastic materials that are different in molecular weights will be described.

- Hydrolysis of Methyl Acetate -

Example 25

To a twin-screw kneader were fed 150 g of sulfonated Polybutene HV 3000 and 150 g of Tetrax 3T and 140 g of water and they were kneaded at a temperature of 40 to 45°C. By this kneading, the above mixture exhibited good spinnability.

16

Then, 15 g of methyl acetate and 160 g of water were added to the kneader and they were further kneaded. The decrease of methyl acetate in the water phase and the increase of methanol produced by hydrolysis were determined so as to examine the rate of hydrolysis. As a result, the rates of hydrolysis at 2, 4, 8 and 12 hours after the addition of the methyl acetate were 31, 47, 83 and 81% by mole, respectively.

Example 26

In place of the 300 g mixture of sulfonated polyisobutylenes in Example 25, a mixture of 225 g of Tetrax 3T and 75 g of sulfonated Polybutene HV 1900 in Experiment C and a mixture of 210 g of Tetrax 6T and 90 g of sulfonated Polybutene HV 300 in Experiment D were used to hydrolyze methyl acetate in the like manner as Example 25. The rates of hydrolysis at 2, 4, 8 and 12 hours after the start of the reaction are shown in the following Table 6.

Table 6

| Time (hrs) | 2 | 4 | 8 | 12 |
|---|---|---|---|---|
| Experiment C | 40 | 55 | 73 | 83 |
| Experiment D | 41 | 59 | 76 | 82 |

Example 27

In place of the 300 g mixture used in Example 25, a mixture of 90 g of sulfonated Polybutene HV 3000, Vistanex MML-80 and 48 g of kerosene as a softening agent were used and kneaded in the like manner as Example 25 in which good spinnability was exhibited. The hydrolysis of methyl acetate was carried out in the like manner as in Example 25. The rates of hydrolysis at 2, 4, 8 and 12 hours after the start of the reaction were 39, 55, 76 and 87% by mole, respectively.

Example 28

In place of the 300 g mixture used in Example 25, 300 g of sulfonated Polybutene HV 3000 was kneaded in the like manner as Example 25, in which good spinnability was not exhibited and the kneading condition was on the very limit where the kneading was possible. The hydrolysis of methyl acetate was carried out in the like manner as in Example 25. The rates of hydrolysis at 2, 4, 8 and 12 hours after the start of reaction were 7, 10, 14 and 15% by mole, respectively. From this results, it was understood that the rate of hydrolysis is better when a mixture of viscoelastic materials is used as compared with the case of a single viscoelastic material.

- Cation Exchange -

Example 29

To a twin-screw kneader were fed a mixture of 150 g of sulfonated Polybutene HV 3000 and 150 g of Tetrax 3T and 140 g of water and they were kneaded at a temperature of 40 to 45°C, in which the mixture exhibited good spinnability.

After that, 50 g of saturated saline solution and 110 g of water were added to the kneader and further kneaded. The concentration of hydrogen ions released into the aqueous phase by the ion exchange was determined by acidimetry to examine the ion exchange efficiency. As a result, the ion exchange rates at 1, 2, 4, and 6 hours after the addition of the saline solution were 47, 74, 83 and 85%, respectively.

The ion exchange rates were expressed by the molar percent of the sulfonic acid groups in the sulfonated Polybutene that were neutralized by the sodium ions.

Example 30

In place of the 300 g mixture of viscoelastic material used in Example 29, a mixture of 225 g of Tetrax 3T and 75 g of sulfonated Polybutene HV 1900 in Experiment E and a mixture of 210 g of Tetrax 6T and 90 g of sulfonated Polybutene HV 300 in Experiment F were used to examine the rates of ion exchange using saline solutions in the like manner as Example 29. The rates of ion exchange at 1, 2, 4 and 6 hours after the addition of the saline solutions are shown in the following Table 7.

Table 7

| Time (hrs) | 1 | 2 | 4 | 6 |
|---|---|---|---|---|
| Experiment E | 28 | 46 | 86 | 94 |
| Experiment F | 65 | 72 | 78 | 83 |

Example 31

In place of the 300 g mixture of viscoelastic material used in Example 29, 300 g of sulfonated Polybutene HV 3000 was kneaded in the like manner as Example 29, in which good spinnability was not exhibited and the kneading condition was on the very limit where the kneading was possible. The ion exchange with saline solution was carried out in the like manner as in Example 29. The rates of hydrolysis at 1, 2, 4 and 6 hours after the addition of the saline solutions were 5, 11, 16 and 19%, respectively. From this results, it was understood that the rate of ion exchange is better when a mixture of viscoelastic materials is used as compared with the case of a single viscoelastic material.

The effect of mixing of the viscoelastic materials will be described again with reference to the hydrolysis of methyl acetate.

Example 32

In place of the 300 g mixture of the viscoelastic material used in Example 10, a mixture of 155 g of sulfonated Tetrax 3T, 75 g of Tetrax 3T, 75 g of Polybutene HV 1900, 10 g of kerosene as a softening agent were used and kneaded in the like manner as Example 10 in which good spinnability was exhibited. The hydrolysis of methyl acetate was carried out in the like manner as in Example 10. The rates of hydrolysis at 2, 4, 8 and 12 hours after the start of reaction were 41, 61, 74 and 78%, respectively.

- Cation Exchange -

Example 33

To a twin-screw kneader were fed a mixture of 150 g of sulfonated Polybutene HV 3000 and 150 g of sulfonated Tetrax 3T and 140 g of water and they were kneaded at a temperature of 40 to 45°C, in which the mixture exhibited good spinnability.

After that, 50 g of saturated saline solution and 110 g of water were added to the kneader and further kneaded. The concentration of hydrogen ions released into the aqueous phase by the ion exchange was determined by acidimetry to examine the ion exchange efficiency. As a result, the ion exchange rates at 1, 2, 4, and 6 hours after the addition of the saline solution were 55, 68, 78 and 88%, respectively.

The ion exchange rates were expressed by the molar percent of the sulfonic acid groups in the sulfonated polyisobutylenes that were neutralized by the sodium ions.

Example 34

In place of the 300 g mixture of viscoelastic material used in Example 33, 300 g of sulfonated Tetrax 3T was used and it was kneaded in the like manner as in Example 33, in which relatively good spinnability was exhibited. The ion exchange efficiency was examined in the like manner as in Example 33. The rates of ion exchange at 1, 2, 4 and 6 hours after the addition of the saline solution were 0, 3, 16 and 45, respectively. It was understood that, when a mixture is used, the ion exchange rates are better.

- Water Holding Capacity under Coexistence of Ions -

Example 35

To a twin-screw kneader were fed 150 g of sulfonated Tetrax 3T and 150 g of Tetrax 3T and they were kneaded at a temperature of 40 to 45°C, to which 5 ml of 1% NaOH aqueous solution (the quantity to neutralize 4% by mole of sulfonic acid groups in the viscoelastic material) was added and further kneaded. After that, the water content was determined in the like manner as in Example 1. The mixture exhibited good spinnability and the water content was 66% by weight.

Example 36

Using 60 mg of $Ca(OH)_2$ (the quantity to neutralize 5% by mole of sulfonic acid groups in the viscoelastic material) was added in place of the NaOH aqueous solution in Example 35 and the water content was determined in the like manner as in Example 1. The mixture exhibited good spinnability and the water content was 68% by weight.

Example 37

To a twin-screw kneader were fed 150 g of sulfonated Tetrax 3T, 150 g of sulfonated Polybutene HV 3000 and 5 g of stearic acid. With kneading it at a temperature of 40 to 45°C, 6 g of water at a time was added succeedingly and it was observed until free water that was not held in the mixture, was released. The mixture exhibited good spinnability. The water content was determined by the point at which the free water appeared. The water content was 120% by weight relative to the mixture of sulfonated polyisobutylenes.

Example 38

In place of 300 g of the mixture of sulfonated polyisobutylenes and the stearic acid which were used in Example 37, the following compositions were used.
(A) 225 g of sulfonated Tetrax 3T, 75 g of sulfonated Polybutene HV 1900 and 10 g of stearic acid.
(B) 115 g of sulfonated Tetrax 3T, 75 g of Tetrax 3T, 75 g of sulfonated Polybutene HV 1900, 10 g of kerosene as a softening agent and 10 g of stearic acid.
(C) 150 g of sulfonated Tetrax 3T, 150 g of sulfonated Polybutene HV 3000 and 15 g of acetic acid.
(D) 225 g of sulfonated Tetrax 3T, 75 g of sulfonated Polybutene HV 1900 and 10 g of acetic acid.
(E) 215 g of Tetrax 6T, 70 g of sulfonated Polybutene HV 3000, 15 g of kerosene as a softening agent and 10 g of phthalic acid.
(F) 150 g of sulfonated Tetrax 6T, 150 g of sulfonated Polybutene HV 1900 and 10 g of rice bran oil (fatty acid triglycerides of carboxylic acids having 16 to 18 carbon atoms).
With regard to the above compositions, water contents were determined in the like manner as in Example 37. All of them exhibited good spinnability and the water contents were (A): 110%, (B): 140%, (C): 90%, (D): 80%, (E): 100% and (F): 120%.

Example 39

Carboxylic acids were removed from the compositions used in Examples 37 and 38 and they were used for determining water contents in the like manner as in Example 37. Even though carboxylic acids were removed, they exhibited good spinnability, however, the water contents of them were all lower than those containing carboxylic acids. The results were as follows:

19

|  | Water Content (%) |
|---|---|
| Experiment corr. to Example 37 | 34 |
| Experiment corr. to Example 38 (A) | 38 |
| " " Example 38 (B) | 48 |
| " " Example 38 (C) | 34 |
| " " Example 38 (D) | 38 |
| " " Example 38 (E) | 36 |
| " " Example 38 (F) | 34 |

- Ion Exchange Function -

Example 40

To a twin-screw kneader were fed a mixture of 150 g of sulfonated Polybutene HV 3000 and 150 g of sulfonated Tetrax 3T and 10 g of stearic acid and 250 g of water. They were kneaded at a temperature of 40 to 45°C for 4 hours, in which the mixture exhibited good spinnability.

After that, 50 g of saturated saline solution and 110 g of water were added to the kneader and further kneaded. The concentration of hydrogen ions released into the aqueous phase by the ion exchange was determined by acidimetry to examine the ion exchange efficiency. As a result, the ion exchange rate at 2 hours after the addition of the saline solution was 67%.

The ion exchange rate was expressed by the molar percent of the sulfonic acid groups contained in the sulfonated polyisobutylene that were neutralized by the sodium ions.

Example 41

In place of the mixture of viscoelastic material used in Example 40, the following compositions were prepared.

(H) 225 g of sulfonated Tetrax 3T, 75 g of sulfonated Polybutene HV 1900 and 10 g of lauric acid.
(I) 225 g of Tetrax 3T, 75 g of sulfonated Polybutene HV 1900 and 10 g of lauric acid.

They were used to examine the rates of ion exchange in the like manner as in Example 40. These compositions exhibited good spinnability. The rates of ion exchange were (H): 68% and (I): 57%, respectively.

Example 42

Carboxylic acids were removed from the compositions used in Examples 40 and 41 and they were used for determining ion exchange efficiency in the like manner as in Example 40. Even though carboxylic acids were removed, they exhibited good spinnability, however, the ion exchange rates of them were all lower than those containing carboxylic acids. The results were as follows:

|  | Ion Exchange Rate |
|---|---|
| Experiment corr. to Example 40 | 55% |
| Experiment corr. to Example 41 (H) | 56% |
| " " Example 41 (I) | 46% |

- Hydrolysis of Carboxylic Acids -

Example 43

To a twin-screw kneader were fed 225 g of Tetrax 3T, 75 g of sulfonated Polybutene HV 1900 and 10 g of stearic acid and they were kneaded together with 200 g of water at a temperature of 40 to 45°C for 4 hours. By this kneading, the above mixture exhibited good spinnability.

Then, 15 g of methyl acetate and 160 g of water were added to the kneader and further kneaded for 2 hours. The methanol produced by hydrolysis in the water phase were determined so as to obtain the rate of hydrolysis. The rate of hydrolysis of methyl acetate was 56% by mole.

Example 44

In place of the 300 g mixture of viscoelastic materials used in Example 43, the following compositions were prepared:

(J) 210 g of Tetrax 6T, 90 g of sulfonated Polybutene HV 300 and 10 g of stearic acid.

(K) 115 g of sulfonated Tetrax 3T, 75 g of Tetrax 3T, 75 g of Polybutene HV 1900, 10 g of kerosene as a softening agent and 10 g of stearic acid.

(L) 90 g of sulfonated Polybutene HV 300, Vistanex MML-80, 48 g of kerosene as a softening agent and 10 g of stearic acid.

With regard to the above compositions, the rates of hydrolysis were determined in the like manner as Example 43. These compositions exhibited good spinnability. The rates of hydrolysis were (J): 53%, (K): 50% and (L): 49%, respectively.

Example 45

To a twin-screw kneader was fed a mixture of 70 g of sulfonated Polybutene HV 3000, 215 g of Tetrax 3T, 15 g of kerosene as a softening agent and 10 g of phthalic acid. They were kneaded together with 250 g of water at a temperature of 40 to 45°C for 4 hours. By this kneading, the above mixture exhibited good spinnability.

Then, 15 g of dimethyl phthalate and 160 g of water were added to the kneader and kneaded for further 2 hours. The methanol produced by hydrolysis in the water phase were determined so as to obtain the rate of hydrolysis. The rate of hydrolysis of dimethyl phthalate was 47% by mole.

Example 46

To a twin-screw kneader was fed a mixture of 150 g of sulfonated Polybutene HV 3000, 150 g of sulfonated Tetrax 3T and 10 g of lauric acid. They were kneaded together with 250 g of water at a temperature of 40 to 45°C for 4 hours. By this kneading, the above mixture exhibited good spinnability.

Then, 15 g of rice bran oil and 160 g of water were added to the kneader and kneaded for further 4 hours. The glycerol produced by hydrolysis in the water phase were determined so as to obtain the rate of hydrolysis. The rate of hydrolysis of rice bran oil was 12% by mole.

Example 47

Carboxylic acids were removed from the compositions used in Examples 43, 44, 45 and 46, and they were used for determining the rates of hydrolysis in the like manner as in Example 43. Even though carboxylic acids were removed, they exhibited good spinnability, however, the rates of hydrolysis of them were all lower than those containing carboxylic acids. The results were as follows:

```
                                   Rate of Hydrolysis

        Experiment corr. to Example 43            46%

        Experiment corr. to Example 44 (J)        43%

              "       "        Example 44 (K)     40%

              "       "        Example 44 (L)     39%

        Experiment corr. to Example 45            41%

        Experiment corr. to Example 46             7%
```

**Claims**

1.  A method for effecting a catalytic chemical reaction by converting a first substance into a second substance which comprises the steps:
    (1) mixing (a) a viscoelastic material having functional groups which exhibit catalytic activity for the reaction, which viscoelastic material is capable of exhibiting spinnability under high shearing stress, and (b) a liquid containing the first substance, which liquid has substantially no affinity for said viscoelastic material,
    (2) kneading the mixture in a kneader at temperatures in the range of 0° to 100°C with the mixture exhibiting spinnability, and
    (3) converting thereby said first substance into said second substance.

2.  A method as claimed in claim 1, wherein said viscoelastic material is an organic polymer.

3.  A method as claimed in claim 1 or claim 2 wherein said functional groups have ion exchange capacity.

4.  A method as claimed in claim 3, wherein the viscoelastic material with functional groups having ion exchange capacity is mixed with water containing ions and they are then kneaded in a kneader, thereby changing said ions contained in said water into other ions by the ion exchange action of said functional groups.

5.  A method as claimed in any one of the preceding claims wherein esters or acetals as said reactants are hydrolyzed.

6.  A method as claimed in any one of claims 1 to 4 wherein acids and alcohols as said reactants are esterified.

7.  A method as claimed in any one claims 1 to 4 wherein aldehydes as said reactants are converted into acetals.

8.  A method as claimed in any one of the preceding claims, wherein the functional groups of said viscoelastic material are sulfonic acid groups and/or carboxylic acid groups or their salts.

9.  A method as claimed in any of the preceding claims, wherein the functional groups of said viscoelastic material are nitrogen-containing functional groups.

10. A method as claimed in claim 9, wherein said nitrogen-containing functional groups are primary to tertiary amino groups or their derivatives.

11. A method as claimed in any one of the claims 2 to 10, wherein said organic polymer is at least one polymer selected from polybutene, polyisobutylene, butyl rubber, polyisoprene, polybutadiene, styrene-butadiene rubber, polychloroprene rubber, acrylonitrile rubber, natural rubber, atactic polypropylene, polystyrene, polyethylene, polyvinyl chloride, polyvinyl acetate, polyvinyl alcohol, polyamide, asphalt, and pitch.

12. A method as claimed in any one of the preceding claims, wherein 40 to 300% by weight, preferably 40 to 200% by weight of said liquid is mixed with said viscoelastic material.

13. A method as claimed in any one of the preceding claims, wherein said organic polymer is a mixture comprising a polymer having no functional group and a number average molecular weight of 1,000 to 5,000 and a polymer having functional groups and a viscosity average molecular weight of 10,000 to 100,000, and said polymer is preferably a polyolefin or a polymer of copolymer of isobutylene.

14. A method as claimed in any one of the preceding claims, wherein the ratio of said functional groups contained in said viscoelastic material is at least 0.005 meq per gram of said viscoelastic material.

**Patentansprüche**

1. Verfahren zum Durchführen einer katalytischen, chemischen Reaktion, wobei eine erste Substanz in eine zweite Substanz umgewandelt wird, welches die folgenden Schritte umfaßt:
   (1) Mischen (a) eines funktionelle Gruppen enthaltenden viskoelastischen Materials, das für die Reaktion katalytisch wirkt und das unter hoher Scherspannung verspinnbar ist und (b) einer Flüssigkeit, die die erste Substanz enthält und die zu dem viskoelastischen Material im wesentlichen keine Affinität zeigt,
   (2) Kneten des Gemisches in einem Kneter bei Temperaturen im Bereich von 0 bis 100 °C, wobei das Gemisch Verspinnbarkeit zeigt, und
   (3) dadurch Umwandeln der ersten Substanz in die zweite Substanz.

2. Verfahren nach Anspruch 1, wobei das viskoelastische Material ein organisches Polymer ist.

3. Verfahren nach Anspruch 1 oder 2, wobei die funktionellen Gruppen Ionenaustausch-Kapazität besitzen.

4. Verfahren nach Anspruch 3, wobei das funktionelle Gruppen enthaltende viskoelastische Material mit Ionenaustausch-Kapazität mit Wasser, das Ionen enthält, gemischt und beide anschließend in einem Kneter geknetet werden, wobei durch die Ionenaustausch-Wirkung der funktionellen Gruppen die in dem Wasser enthaltenen Ionen durch andere Ionen ausgetauscht werden.

5. Verfahren nach einem der vorhergehenden Ansprüche, wobei als die Reaktanten Ester oder Acetale hydrolysiert werden.

6. Verfahren nach einem der Ansprüche 1 bis 4, wobei als die Reaktanten Säuren und Alkohole verestert werden.

7. Verfahren nach einem der Ansprüche 1 bis 4, wobei als die Reaktanten Aldehyde in Acetale umgewandelt werden.

8. Verfahren nach einem der vorhergehenden Ansprüche, wobei die funktionellen Gruppen des viskoelastischen Materials Sulfonsäuregruppen und/oder Carbonsäuregruppen oder ihre Salze sind.

9. Verfahren nach einem der vorhergehenden Ansprüche, wobei die funktionellen Gruppen des viskoelastischen Materials Stickstoff enthaltende funktionelle Gruppen sind.

10. Verfahren nach Anspruch 9, wobei die Stickstoff enthaltenden funktionellen Gruppen primäre bis tertiäre Aminogruppen oder ihre Derivate sind.

11. Verfahren nach einem der Ansprüche 2 bis 10, wobei das organische Polymer mindestens ein unter Polybuten, Polyisobutylen, Butyl-Kautschuk, Polyisopren, Polybutadien, Styrol-Butadien-Kautschuk, Polychloropren-Kautschuk, Acrylnitril-Kautschuk, natürlichem Kautschuk, ataktischem Polypropylen, Polystyrol, Polyethylen, Polyvinylchlorid, Polyvinylacetat, Polyvinylalkohol, Polyamid, Asphalt und Pech ausgewähltes Polymer ist.

**12.** Verfahren nach einem der vorhergehenden Ansprüche, wobei 40 bis 300 Gew.%, vorzugsweise 40 bis 200 Gew.% der Flüssigkeit mit dem viskoelastischen Material gemischt werden.

**13.** Verfahren nach einem der vorhergehenden Ansprüche, wobei das organische Polymer ein Gemisch ist, das ein Polymer ohne funktionelle Gruppen mit einem Molekulargewichtszahlenmittel von 1 000 bis 5 000 und ein Polymer mit funktionellen Gruppen und mit einem Viskositätsmittel des Molekulargewichts von 10 000 bis 100 000 enthält, wobei dieses Polymer vorzugsweise ein Polyolefin oder ein Polymer oder Copolymer von Isobutylen ist.

**14.** Verfahren nach einem der vorhergehenden Ansprüche, wobei der Anteil der in dem viskoelastischen Material enthaltenen funktionellen Gruppen mindestens 0,005 meq/g des viskoelastischen Materials ist.

**Revendications**

**1.** Procédé pour effectuer une réaction chimique catalytique en transformant une première substance en une deuxième substance, qui comporte les étapes consistant à :
(1) mélanger (a) un matériau viscoélastique comportant des groupes fonctionnels qui présentent une activité catalytique pour la réaction, lequel matériau viscoélastique étant susceptible de filage quand il est soumis à une contrainte de cisaillement élevée, et (b) un liquide contenant la première substance, lequel liquide ne présente sensiblement pas d'affinité pour ledit matériau viscoélastique,
(2) malaxer le mélange dans un malaxeur à des températures comprises entre 0° et 100°C, de sorte que le mélange soit susceptible de filage, et
(3) transformer ainsi ladite première substance en ladite deuxième substance.

**2.** Procédé selon la revendication 1, dans lequel ledit matériau viscoélastique est un polymère organique.

**3.** Procédé selon la revendication 1 ou 2, dans lequel lesdits groupes fonctionnels présentent une capacité d'échange ionique.

**4.** Procédé selon la revendication 3, dans lequel le matériau viscoélastique dont les groupes fonctionnels ont une capacité d'échange ionique est mélangé avec de l'eau contenant des ions et ils sont ensuite malaxés dans un malaxeur, transformant ainsi lesdits ions contenus dans ladite eau en d'autres ions par l'action d'échange ionique desdits groupes fonctionnels.

**5.** Procédé selon l'une quelconque des revendications précédentes, dans lequel lesdits réactifs, lorsque ce sont des esters ou des acétals, sont hydrolysés.

**6.** Procédé selon l'une quelconque des revendications 1 à 4, dans lequel lesdits réactifs, lorsque ce sont des acides et des alcools, sont estérifiés.

**7.** Procédé selon l'une quelconque des revendications 1 à 4, dans lequel lesdits réactifs, lorsque ce sont des aldéhydes, sont transformés en acétals.

**8.** Procédé selon l'une quelconque des revendications précédentes, dans lequel les groupes fonctionnels dudit matériau viscoélastique sont des groupes acide sulfonique et/ou des groupes acide carboxylique ou leurs sels.

**9.** Procédé selon l'une quelconque des revendications précédentes, dans lequel les groupes fonctionnels dudit matériau viscoélastique sont des groupes fonctionnels contenant de l'azote.

**10.** Procédé selon la revendication 9, dans lequel lesdits groupes fonctionnels contenant de l'azote sont des groupes amino primaires à tertiaires ou leurs dérivés.

**11.** Procédé selon l'une quelconque des revendications 2 à 10, dans lequel ledit polymère organique est au moins un polymère choisi parmi les polybutène, polyisobutylène, caoutchouc butyle, polyisoprène, polybutadiène, caoutchouc styrène-butadiène, caoutchouc polychloroprène, caoutchouc acrylonitrile, caoutchouc naturel, polypropylène atactique, polystyrène, polyéthylène, chlorure de polyvinyle, acétate de polyvinyle, alcool polyvinylique, polyamide, asphalte et poix.

**12.** Procédé selon l'une quelconque des revendications précédentes, dans lequel on mélange 40 à 300 % en poids, de préférence 40 à 200 % en poids dudit liquide avec ledit matériau viscoélastique.

**13.** Procédé selon l'une quelconque des revendications précédentes, dans lequel ledit polymère organique est un mélange comprenant un polymère n'ayant pas de groupe fonctionnel et un poids moléculaire moyen en nombre de 1000 à 5 000 et un polymère ayant des groupes fonctionnels et un poids moléculaire moyen viscosimétrique de 10 000 à 100 000, et ledit polymère est de préférence une polyoléfine ou un polymère de copolymère d'isobutylène.

**14.** Procédé selon l'une quelconque des revendications précédentes, dans lequel la proportion desdits groupes fonctionnels contenus dans ledit matériau viscoélastique est au moins de 0,005 meq par gramme dudit matériau viscoélastique.